(19) Europäisches Patentamt
European Patent Office
Office européen des brevets

(11) Veröffentlichungsnummer : **0 248 312 B1**

(12) # EUROPÄISCHE PATENTSCHRIFT

(45) Veröffentlichungstag der Patentschrift : **27.03.91 Patentblatt 91/13**

(51) Int. Cl.[5] : **C07D 277/82**, C09B 29/045, C12Q 1/28, G01N 33/72

(21) Anmeldenummer : **87107582.6**

(22) Anmeldetag : **25.05.87**

(54) **4,6-Dinitrobenzthiazolon-hydrazone (2).**

Die Akte enthält technische Angaben, die nach dem Eingang der Anmeldung eingereicht wurden und die nicht in dieser Patentschrift enthalten sind.

(30) Priorität : **04.06.86 DE 3618752**

(43) Veröffentlichungstag der Anmeldung : **09.12.87 Patentblatt 87/50**

(45) Bekanntmachung des Hinweises auf die Patenterteilung : **27.03.91 Patentblatt 91/13**

(84) Benannte Vertragsstaaten : **DE FR GB IT**

(56) Entgegenhaltungen :
**EP-A- 0 068 356**
**EP-A- 0 071 730**
**US-A- 3 057 848**
**US-A- 4 119 405**
**CHEMICAL ABSTRACTS, Band 86, Nr. 3, 17. Januar 1977, Columbus, OH (US); D.RAMAMURTHY et al., S. 420, Nr. 16606p**

(73) Patentinhaber : **BAYER AG**
**W-5090 Leverkusen 1 Bayerwerk (DE)**

(72) Erfinder : **Hugl, Herbert, Dr.**
**Gemarkenweg 9**
**W-5060 Bergisch-Gladbach 2 (DE)**
Erfinder : **Engel, Aloysius, Dr.**
**Am Brungen 17**
**W-5090 Leverkusen 3 (DE)**
Erfinder : **Wehling, Klaus, Dr.**
**Am Rohm 121**
**W-5600 Wuppertal 1 (DE)**

**EP 0 248 312 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es wurde gefunden, daß sich 4,6-Dinitrobenzthiazolonhydrazone(2) zur Herstellung wertvoller Azofarbstoffe eignen und ferner als Farbbildner beim Nachweis von Substanzen, insbesondere bei der Bestimmung von $H_2O_2$, eingesetzt werden können.

Gegenstand der vorliegenden Erfindung sind 4,6-Dinitrobenzthiazolonhydrazone(2) der Formel (I)

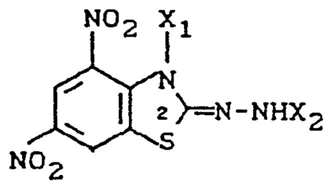

(I)

Worin

$X_1$ für Wasserstoff, $C_1$-$C_4$-Alkyl, $C_1$-$C_4$-Hydroxyalkyl, $C_1$-$C_4$-Sulfoalkyl oder $C_1$-$C_4$-Sulfatoalkyl,

$X_2$ für Wasserstoff steht.

Vorzugsweise steht $X_1$ für Wasserstoff oder die Methylgruppe. Vorzugsweise steht $X_1$ auch für eine Doppelbindung zwischen dem cyclischen Stickstoffatom und dem Kohlenstoffatom 2 gemäß nachstehender Formel II :

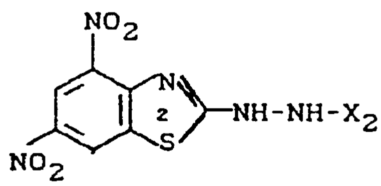

(II)

wobei $X_2$ die unter der allgemeinen Formel I angegebene Bedeutung hat.

Die US-A-3 057 848 beschreibt 2-Amino-4,6-dinitrobenzthiazole und derar Verwendung zur Herstellung von Azofarbstoffen un deren Verwendung als Färbemittel.

Die US-A-4 119 405 und die EP-A- 68356 beschreiben MBTH und dessen Einsatz in der medizinischen Diagnostik zusammen mit verschiedenen Kupplungs-Komponenten.

Ein weiterer Gegenstand der vorliegenden Erfindung ist die Verwendung von 4,6-Dinitrobenztbiazolonhydrazonen(2) der allgemeinen Formel I zur Herstellung von Azofarbstoffen und als Farbbildner beim Nachweis von Substanzen, insbesondere bei der Bestimmung von $H_2O_2$. Unter Farbbildner im Sinne der vorliegenden Erfindung werden solche Substanzen verstanden, die nach Oxidation mit einer geeigneten Kupplungskomponente einen Farbstoff bilden. Die vorliegende Erfindung betrifft daher auch allgemein das Gebiet von diagnostischen Tests und hier insbesondere solche Tests, welche die qualitative und quantitative Bestimmung von oxidierbaren biologischen Substanzen, z. B. Milchsäure, Glucose, Cholesterin, Harnsäure, Kreatinin, GPT oder auch Glycerin und Ketokörper, betreffen. In solchen Tests werden diese biologiscben Substanzen üblicherweise durch eine spezifische Oxidase oxidiert. Durch die katalytische Wirkung einer Peroxidase oder peroxidativ wirksamen Substanz, wie z.B. Hämoglobin oder Methhämoglobin erfolgt dann eine $H_2O_2$-abhängige Oxidation des erfindungsgemäßen Farbbildners. Das Oxidationsprodukt ist dann in der Lage mit einer geeigneten Kupplungkomponente unter Bildung eines Farbstoffs zu reagieren. Ebenso sind die erfindungsgemäßen 4,6-Dinitrobenzthiazolonhydrazone (2) hervorragend zum Nachweis von Peroxidase oder einer peroxidativ wirksamen Substanz geeignet. Ein solcher Nachweis erfolgt unter Ausnutzung der vorstehend beschriebenen Oxidations- und Kupplungsreaktionen, wobei als Oxidationsmittel $H_2O_2$ oder ein anderes Peroxid, z.B. Cumolbydroperoxid, Strontiumperoxid oder 2,5-Dimethylhexan-2,5-dihydroperoxid eingesetzt werden.

Ein weiterer Bestandteil der vorliegenden Erfindung besteht darin, daß entweder in Lösung oder in einer Vorrichtung, z. B. als Reagenz, in einem Teststreifen oder einem analytischen Element die erfindungegemäßen Farbbildner 4,6-binitrobenzthiazolonhydrazone(2) gemäß allgemeiner Formel (I) eine sichtbare Farbänderung der zu untersuchenden Probe bewirken.

4,6-Dinitrobenzthiazolonhydrazone(2) können nach folgendem Verfahren mit guten Ausbeuten gewonnen werden.

Durch Nitrierung von 2-Amino-benzthiazol erhaltenes 2-Amino-4,6-dinitrobenzthiazol wird in einem geeigneten Lösungsmittel mit Hydrazinhydrat umgesetzt. Als Lösungsmittel wird beispielsweise 2-Ethoxyethanol eingesetzt. Vorzugsweise erfolgt die Umsetzung unter Wärmezufuhr, besonders bevorzugt bei 70°C bis 150°C,

hervorragende Ausbeuten ergibt das Arbeiten beim Kochpunkt der Mischung (ca. 128-130°C).

Hydrazinhydrat wird bevorzugt 100%ig eingesetzt. Die Reaktionsdauer beträgt ca. 1 Stunde, eine kontinuierliche Verfahrensweise ist auch möglich. Das Reaktionsgemisch wird nach Abkühlen in eine angesäuerte, wäßrige Lösung gegeben. Gut einsetzbar ist eine wäßrige Eisessiglösung. Das Reaktionsprodukt wird abgetrennt, vorzugsweise gewaschen und getrocknet.

Es wurde gefunden, daß die neuen 4,6-Dinitrobenzthiazolonhydrazone(2) oxidiert und dann mit einer Vielzahl von Kupplungskomponenten zur Herstellung von Azofarbstoffen verwendet werden können. Solchermäßen hergestellte Azofarbstoffe sind besonders gut bei der Färbung von natürlichen und Kunstfasern, Geweben oder Gewirken einsetzbar.

Die Herstellung von Azofarbstoffen aus 4,6-Dinitrobenzthiazolonhydrazonen(2) durch oxidative Kupplung stellt ein einfaches und wirtschaftlich sehr interessantes Verfahren dar.

Als Kupplungskomponenten für die oxydative Kupplung können einem elektrophilen Angriff zugängliche Aromaten, wie z.B. die nachstehenden Verbindungen erfindungsgemäß eingesetzt werden :

Kupplungskomponenten

1.

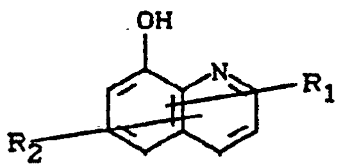

$R_1$ und $R_2$ können gleich oder verschieden sein und bedeuten Wasserstoff, $C_1$-$C_4$-Alkyl, $C_1$-$C_4$-Alkoxy, Phenyl, Fluor, Chlor, Brom, Iod, bevorzugt Wasserstoff.

2.

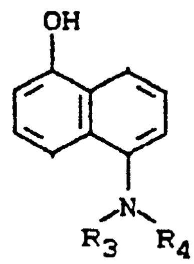

$R_3$ und $R_4$ können gleich oder verschieden sein und bedeuten Wasserstoff, $-C_2H_4$-O-$R_5$, $-C_2H_4$-O-CO-$R_5$, $C_1$-$C_4$-Alkyl, wobei $R_5$ für $C_1$-$C_4$-Alkyl oder Phenyl steht, vorzugsweise Wasserstoff.

3.

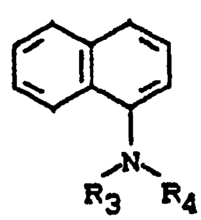

$R_3$ und $R_4$ haben die gleiche Bedeutung wie unter 2. angegeben, vorzugsweise Wasserstoff oder Ethyl.

4.

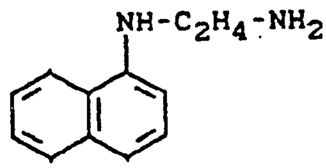

5.

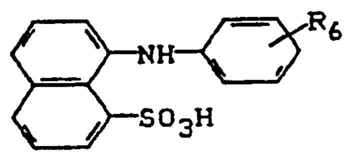

$R_6$ bedeutet Wasserstoff oder Methyl.

6.

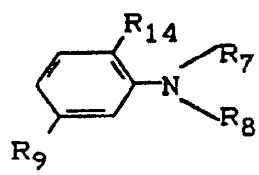

$R_7$ und $R_8$ können gleich oder verschieden sein und bedeuten : Wasserstoff, gegebenenfalls durch Sulfo, Sulfato, Hydroxy, Cyan, Halogen, $C_1$-$C_4$-Alkoxy, $-OCOR_5$, $-COOR_5$, $OCONHR_5$, Phenyl oder Sulfophenyl substituiertes $C_1$-$C_8$-Alkyl, sowie $C_3$-$C_6$-Alkenyl. $R_5$ steht dabei für $C_1$-$C_4$-Alkyl oder Phenyl, Vorzugsweise bedeuten $R_7$ und $R_8$ Wasserstoff, Ethyl, Hydroxyethyl, Cyanethyl, Propyl, Butyl, Acetoxyethyl, Methoxycarbonylethyl, Ethoxycarbonylethyl, Methylaminocarbonyloxyethyl, Phenylaminocarbonyloxyethyl, Benzyl, Phenethyl sowie Sulfobenzyl.

$R_9$ bedeutet Wasserstoff $C_1$-$C_4$-Alkyl, $C_1$-$C_4$Alkoxy, Hydroxy, $-NHCOR_5$, $-NHSO_2R_5$, wobei $R_5$ die oben angegebene Bedeutung hat.

Vorzugsweise bedeutet $R_9$ Wasserstoff, Methyl, $NHCOCH_3$ und $NHSO_2CH_3$.

$R_{14}$ bedeutet Wasserstoff, Halogen, $C_1$-$C_4$-Alkyl, $C_1$-$C_4$-Alkoxy oder $C_2$-$C_8$-Alkoxyalkyl, bevorzugt Wasserstoff, Chlor, Methyl oder Methoxy.

7.

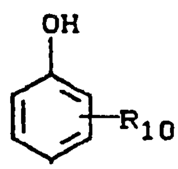

$R_{10}$ bedeutet Wasserstoff, Methyl, Hydroxy, Chlor, vorzugsweise Methyl oder Hydroxy.

8.

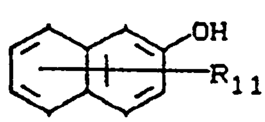

$R_{11}$ bedeutet Wasserstoff, COOH oder $SO_3H$, vorzugsweise

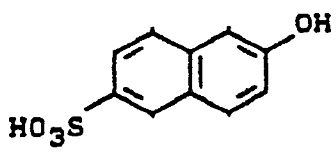

oder

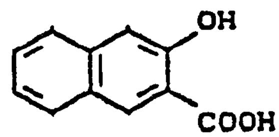

9.

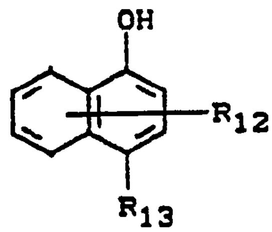

$R_{12}$ bedeutet Wasserstoff oder $SO_3H$, vorzugsweise Wasserstoff und
$R_{13}$ bedeutet Wasserstoff oder $O\text{-}CH(CH_3)\text{-}COOH$.

10.

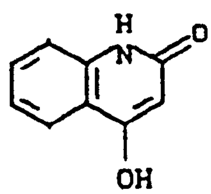

11.

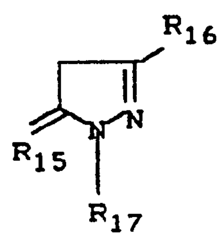

$R_{15}$ bedeutet O, NH,
$R_{16}$ bedeutet $CH_3$, COOH, $NH_2$, vorzugsweise Methyl
$R_{17}$ bedeutet $C_2$-$C_8$-Alkyl, H, Phenyl, Sulfophenyl, vorzugsweise Phenyl oder Sulfophenyl.

12.

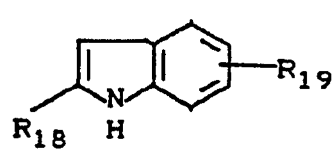

$R_{18}$ bedeutet $CH_3$, Phenyl, vorzugsweise Methyl und
$R_{19}$ bedeutet H, $SO_3H$.

13.

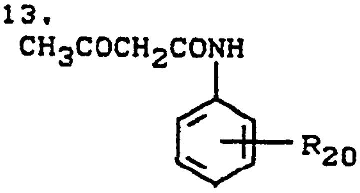

$R_{20}$ bedeutet H, $OCH_3$, Cl, $CH_3$, vorzugsweise $OCH_3$.

14.

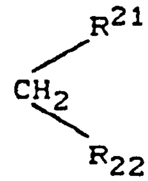

$R_{21}$, $R_{22}$ können gleich oder verschieden sein und bedeuten CN, $COOC_2H_5$, vorzugsweise CN.

15.

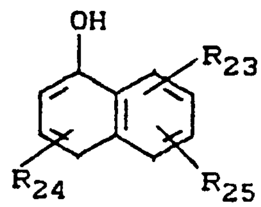

$R_{23}$ bedeutet $NH_2$, OH,
$R_{24}$ bedeutet Wasserstoff, $SO_3H$ und
$R_{25}$ bedeutet H, $SO_3H$, vorzugsweise

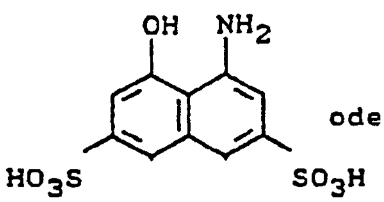

oder

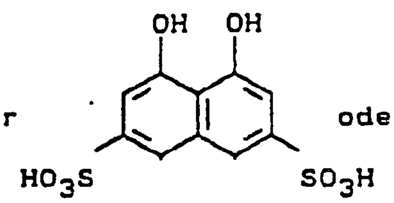

oder

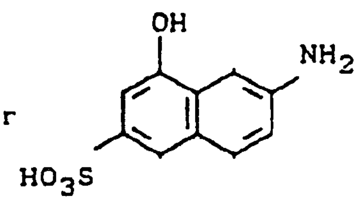

16.

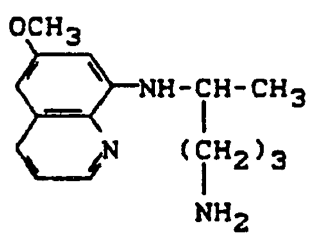

Alle genannten Kupplungskomponenten sind sowohl für die Herstellung von Azofarbstoffen zum Färben von textilen Fasern als auch für die Durchführung diagnostischer Tests nach dem erfindungsgemäßen Verfahren wertvoll. Ganz besonders hochwertige Farbstoffe zum Färben von Kunstfasern erhält man bei Verwendung der Kupplungskomponenten der Formel wie vorstehend unter 6. angegeben.

Die oxidative Kupplung zur Herstellung von Textilfarbstoffen wird in an sich bekannter Weise durchgeführt. Als Oxidationsmittel finden anorganische Salze wie Periodate, Borate, Ferrate und Peroxodisulfate Verwendung : besonders geeignet sind Natriummetaperiodat, Kaliumhexacyanoferrat und Kaliumbromat.

Die so erhaltenen Farbstoffe sind auch über Diazotierung von 2-Amino-4,6-dinitrobenzthiazol und Kupplung zugänglich, beispielsweise nach dem in der US-Patentschrift 3 057 848 angegebenen Verfahren.

Die vorliegende Erfindung betrifft ferner ein Verfahren zur Bestimmung von oxidierbaren biologischen Substanzen. Hierunter werden im Sinne der vorliegenden Erfindung solche Substanzen verstanden, die unter Erzeugung oder Freisetzung von $H_2O_2$ oxidiert werden. Diese Reaktion läuft vorzugsweise in wäßrigem Medium ab.

Zur Durchführung dieses Nachweisverfahrens wird eine Probe der zu analysierenden oxydierbaren biologischen Substanz mit einer geeigneten Oxidase und einer Substanz mit peroxidativer Aktivität, den erfindungsgemäßen 4,6-Dinitrobenzthiazolonhydrazonen(2) als Farbbildner sowie einer Kupplungskomponente in

Kontakt gebracht, Des weiteren betrifft die Erfindung analytische Elemente sowie Reagenzien zur Durchführung dieses Verfahrens ; gegebenenfalls können solche Reagenzien weitere Substanzen wie z. B. Puffer, Netzmittel und Stabilisatoren enthalten. Geeignete Puffer sind Borat, Citrat, Phosphat, Glutarat, Carbonat oder Tris-Puffer. Die damit eingestellten pH-Werte liegen bei 5 bis 8, vorzugsweise 7 bis 8.

Die qualitative Erkennbarkeit und quantitative Bestimmbarkeil von $H_2O_2$ sowie Substanzen, die unter Erzeugung oder Freisetzung von $H_2O_2$ reagieren, ist auf vielen Gebieten sehr wichtig. Beispielsweise genannt sei die Erkennung und Bestimmung von $H_2O_2$, das bei der enzymatischen Bestimmung von Substanzen, z. B. Glucose, Cholesterin, Harnsäure und vielen anderen mehr durch die Aktivität von Enzymen, beispielsweise Glucose-Oxidase, Cholesterin-Oxidase und Uricase in Gegenwart von Sauerstoff gebildet wird. Das Vorkommen oder sogar die quantitative Menge eines in einer Probe vorhandenen enzym-Substrates läßt sich aus der Menge an erzeugtem $H_2O_2$ ermitteln.

Bekannte Reagenzien für die Erkennung und/oder quantitative Bestimmung von $H_2O_2$, das in solchen Systemen erzeugt wird, enthalten im allgemeinen eine Substanz mit peroxidativer Aktivität, beispielsweise Peroxidase oder peroxidaseartige Substanzen wie z.B. Hämoglobin sowie ferner eine Substanz, die in Gegenwart von $H_2O_2$ einer erkennbaren Veränderung unterliegt. Peroxidase ist ein Enzym, welches die Oxidation des Farbbildners durch $H_2O_2$ katalysiert. Die Gewinnung von Peroxidasen ist allgemein bekannt, solche enzyme sind im Handel erhältlich. Die oxydative Kupplung zum Nachweis von $H_2O_2$ ist bereits bekannt, vgl. H. U. Bergmeyer, Methods of Enzymatic Analysis, 3rd Ed, Vol I, 1984, S. 216. Überraschenderweise wurde gefunden, daß sich 4,6-Dinitrobenzthiazolonhydrazone(2) sehr gut als Farbbildner in einer solchen Reaktion eignet.

Für die Durchführung einer solchen Nachweisreaktion ist das Vorliegen oder die Bildung von $H_2O_2$, der Substanz mit peroxidativer Aktivität und dem Farbbildner sowie Farbkuppler wichtig. Diese Mischung bewirkt die Änderung der Farbe in der zu untersuchenden Probe.

Die vorliegende Erfindung gestattet die Ermittlung von sehr geringen Konzentrationen an $H_2O_2$. Eingesetzt wird vorzugsweise ein abgepuffertes Reagenz oder Reagenz-system mit einer bestimmten Zusammensetzung. Vorteilhafterweise ist die Farbreaktion sehr empfindlich und auch über einen großen Konzentrationsbereich linear, so daß in diesem Bereich durch einfaches Ablesen von z.B. Reagenzstreifen zumindest halbquantitative Aussagen gemacht werden können. Der immense Vorteil der vorliegenden Erfindung wird hierdurch anschaulich verdeutlicht. Der gebildete Farbton ist sehr lange farbstabil und gestattet eine Ablesung über einen weiten Zeitbereich. Durch Kombination verschiedener Kupplungskomponenten lassen sich ferner Färbungen in unterschiedlichen Nuancen erzielen. Insbesondere gilt dies für den langwelligen Bereich, für den bisher nur wenige Chromogene zur Verfügung stehen. Dadurch können spektrale Interferenzen mit Hämoglobin und Bilirubin oder Trübungseinflüsse weitgehend vermieden werden.

Veranschaulicht liegt der Farbreaktion zum Nacbweis von $H_2O_2$ oder Substanzen, die $H_2O_2$ freisetzen, folgendes Reaktionsschema zugrunde :

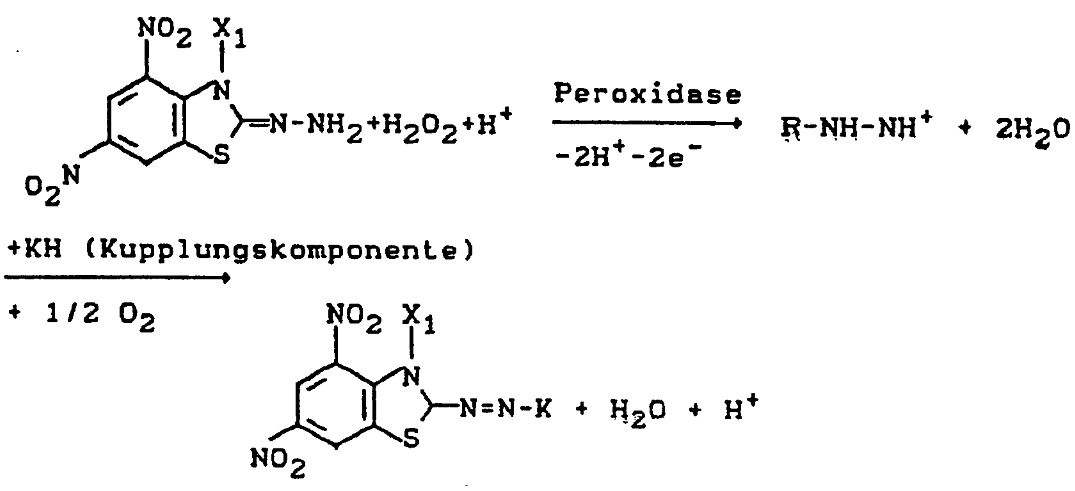


Die Bestimmung der Farbstoffdichte als quantitativer Meßwert für die Konzentration von $H_2O_2$ erfolgt nach üblichen Methoden, z. B. optisch durch Spektrophotometer, vorzugsweise Reflektometer oder durch Vergleich mit Farbskalen, Farbkarten oder mit Standardlösungen.

Die erfindungegemäßen Zusammensetzungen können ferner noch Puffer oder Substanzen, die eine Einstellung eines sauren pH-Bereiches ermöglichen, enthalten. Die Einstellung eines solchen sauren pH-Bereiches ist nicht notwendigerweise vorzunehmen, insbesondere bei der erfindungsgemäßen Verwendung als diagnostisches Reagenz oder Element aber bevorzugt ; eine saure Einstellung ist für die gleichfalls erfindungsgemäße Verwendung zur Herstellung von Azofarbstoffen nicht notwendig.

Die erfindungsgemäßen Reagenzien können in Form von Lösungen vorliegen, denen nur noch die zu untersuchende Probe zugesetzt werden muß oder in Form eines Test-Kits, der kurz vor Bestimmung der Probe angesetzt wird.

In einer weiteren vorteilhaften Ausgestaltung der Erfindung werden analytische Elemente eingesetzt. Solche Elemente lassen sich z. B. durch Imprägnierung geeigneter absorbierender Stoffe, z. B. saugender Papiere und Lösungen der Reagenzien herstellen.

Solche Elemente können auch aus mehreren verschiedenen Schichten bestehen. Die bekannten Methoden zur Herstellung von Teststreifen können bei der vorliegenden Erfindung breit eingesetzt werden.

Die erfindungsgemäßen Bestimmungsreagenzien können auch in Form von trockenen Mischungen vorliegen, die z. B. durch Zusatz von Wasser in diagnostische Lösungen überführt werden können.

Der Gegenstand der vorliegenden Erfindung soll durch die nachfolgenden Beispiele noch näher veranschaulicht werden.

Beispiel 1

Herstellung des Hydrazins der Formel

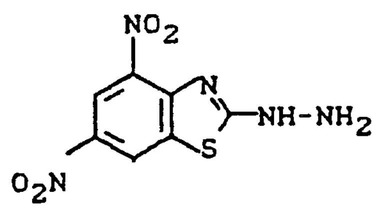

24,0 g Amino-4,6-dinitrobenzthiazol werden in 200 ml 2-Ethoxyethanol mit 20 ml Hydrazinhydrat (100%) 1 Stunde zum Sieden erhitzt. Nach Abkühlen auf Raumtemperatur gießt man in eine Mischung aus 750 ml Wasser und 20 ml Eisessig, rührt 1 Stunde, saugt ab, wäscht mit Wasser und trocknet. Es werden 21,0 g 2-Hydrazino-4,6-dinitrobenzthiazol erhalten. Fp. 210-214°C (Zers.). Nach Umkristallisation aus 2-Methoxyethanol, Fp. 216-217°C (Zers.).

Biespiel 2

Zu 500 ml Eisessig gibt man unter Rühren bei Raumtemperatur innerhalb 4 Stunden in 10 Portionen insgesamt 18,5 g 2-Hydrazino-4,6-dinitrobenzthiazol, 15,0 g N,N-Dibutylanilin und 15,5 g Natriummetaperiodat. Anschließend rührt man 5 Stunden nach, versetzt mit Wasser, saugt ab, kristallisiert aus Dimethylformamid um und trocknet. Der Farbstoff besitzt die Struktur

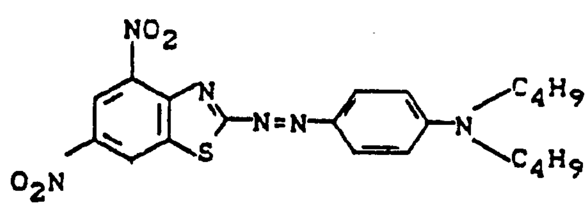

und färbt Polyesterfasern blau mit guten Echtheiten.

Bei analoger oder ähnlicher Arbeitsweise können die in nachfolgender Tabelle aufgeführten Farbstoffe in guter Ausbeute erhalten werden :

Farbstoffe der Formel

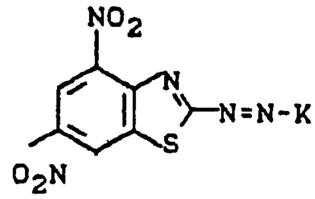

| Beispiel Nr. | K | Nuance auf Polyester |
|---|---|---|
| 3 | 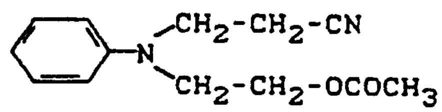 | Rotviolett |
| 4 | 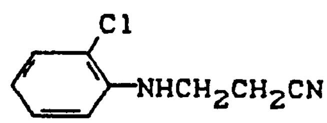 | Rotbordo |
| 5 | 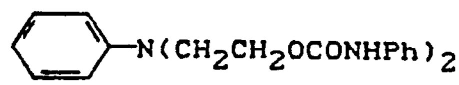 | blaustichig Bordo |
| 6 | 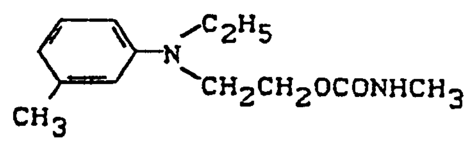 | rotstichig Dunkelblau |

Optische Eigenschaften der Reagenzien zum Nachweis von Peroxid

Zur Ermittlung der optischen Eigenschaften der Peroxidindikatoren wird wie folgt vorgegangen :

4,6-Dinitrobenzthiazolonhydrazone (2) und die entsprechenden Kupplungskomponenten werden als konzentrierte Stammlösungen (2-10 mmol/l) in 0.1 m Puffer angesetzt. Löst sich eine Substanz nicht quantitativ auf, wird mit Puffer/DMF oder Puffer/DMSO Gemischen gelöst. Diese Lösungen werden anschließend gemischt, mit der erforderlichen Pufferlösung weiter verdünnt und der pH-Wert kontrolliert. 500 µl dieser Lösung werden in einer Kuvette mit 10 µl Peroxidase versetzt, die Extinktion des Leerwertes gemessen und danach durch Zugabe von 10 µl frisch hergestellter Peroxidlösung (1-5 mmol/l) die Reaktion gestartet. Nach 5 Minuten wird erneut die Extinktion gemessen und der Leerwert davon abgezogen.

Die eingesetzte Peroxidkonzentration wird durch Messung der Extinktion bei 240 nm kontrolliert. (5 mmol/l $H_2O_2 \hat{=} 0{,}25\ E_{240}$). Für die nachfolgend beschriebenen Indikatoren wurden folgende Konzentrationen im Testansatz werwendet :

| | |
|---|---|
| 4,6-dinitrobenzthiazolonhydrazon | 5 mmol/l |
| Kupplungskomponente | 5 mmol.l |
| Peroxidase | 5 kU/l |
| Citrat-Puffer, pH 5.0 | 100 mmol/l |
| Peroxid-Testlösung : | 5 mmol/l |

Nach 5 Minuten Reaktionszeit wurden folgende Extinktionsdifferenzen bei den angegebenen Absorptionsmaxima gemessen :

| Beispiel Nr. | K | λmax[nm] | ΔE |
|---|---|---|---|
| 7 | 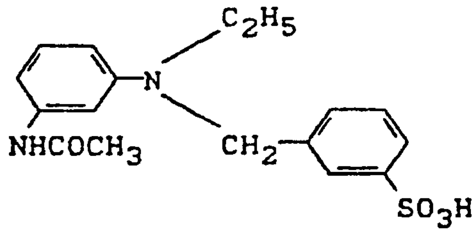 | 570 | 1,393 |
| 8 | 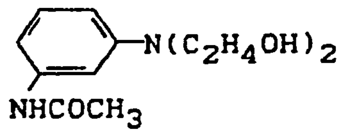 | 590 | 1,573 |
| 9 | 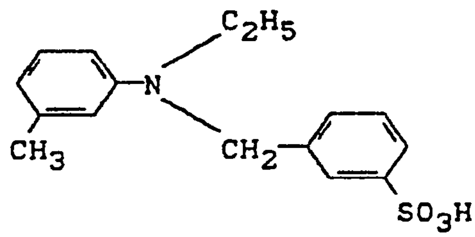 | 615 | 1,638 |
| 10 | 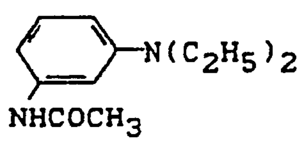 | 585 | 0,834 |
| 11 | 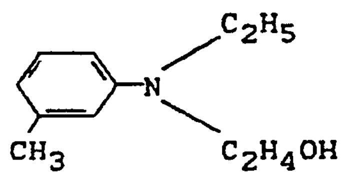 | 615 | 1,841 |

| Beispiel Nr. | K | λmax[nm] | ΔE |
|---|---|---|---|
| 12 | 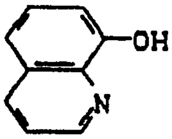 | 590 | 1,700 |
| 13 | 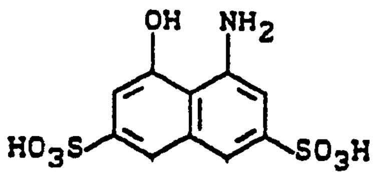 | 554 | 0,400 |
| 14 | 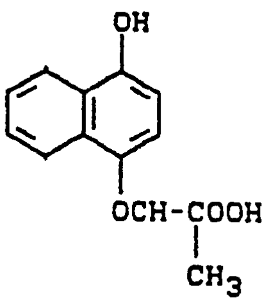 | 620 | 0,200 |
| 15 | 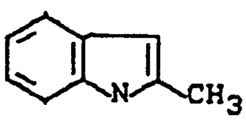 | 510 | 2,240 |
| 16 | 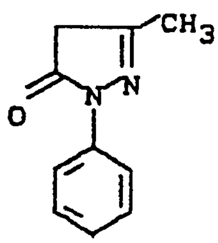 | 508 | 0,065 |

| Beispiel Nr. | K | λmax[nm] | ΔE |
|---|---|---|---|
| 17 | 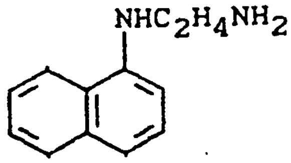 | 508 | 5,200 |
| 18 | 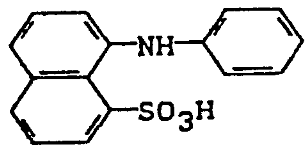 | 658 | 13,500 |
| 19 | 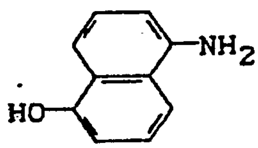 | 580 | 0,390 |
| 20 | 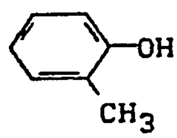 | 592 | 0,100 |

## Ansprüche

1. 4,6-Dinitrobenzthiazolonhydrazone (2) der Formel (I)

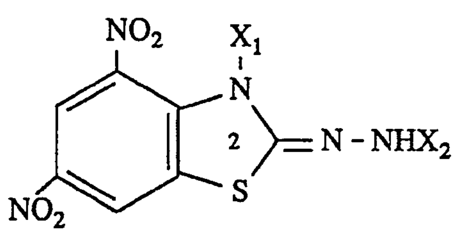

(I)

worin

$X_1$ für Wasserstoff, $C_1$-$C_4$-Alkyl, $C_1$-$C_4$-Hydroxyalkyl, $C_1$-$C_4$-Sulfoalkyl oder $C_1$-$C_4$-Sulfatoalkyl, und $X_2$ für Wasserstoff steht.

2. 4,6-Dinitrobenzthiazolonhydrazone(2) gemäß Anspruch 1, worin $X_1$ für Wasserstoff oder eine Methylgruppe steht.

3. 4,6-Dinitrobenzthiazolonhydrazone(2) gemäß Anspruch 1 der Formel (II)

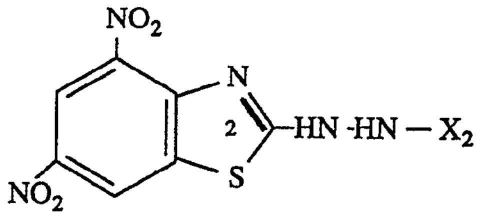

(II)

worin $X_2$ die in Anspruch 1 gegebene Bedeutung hat.

4. Verwendung von 4,6-Dinitrobenzthiazolonhydrazonen(2) gemäß den Ansprüchen 1-3 als Farbbildner bei der Bestimmung von Wasserstoffperoxid.

5. Reagenz für die Bestimmung von Wasserstoffperoxid in wäßrigen Lösungen, enthaltend eine Puffersubstanz, eine Substanz mit peroxidativer Aktivität und 4,6-Dinitrobenzthiazolonhydrazone(2) gemäß den Ansprüchen 1-3 als Farbbildner sowie eine Kupplungskomponente.

6. Analytisches Element für die Bestimmung eines Analyten in einer wäßrigen Flüssigkeit, mit einer nichtfasrigen, isotrop porösen Ausbreitschicht und einer Reagenzschicht und einem Gehalt an

a) einem Enzym, das bei Kontakt mit dem Analyten die Erzeugung von Wasserstoffperoxid bewirkt und

b) einem Reagenz zur Bestimmung von Wasserstoffperoxid bestehend aus

I. einer Substanz mit peroxidativer Aktivität,

II. einem Farbbildner

III. einer Kupplungskomponente, und gegebenenfalls

IV. einer Puffersubstanz, dadurch gekennzeichnet, daß es als Farbbildner 4,6-Dinitrobenzthiazolonhydrazone(2) gemäß den Ansprüchen 1-3 enthält.

7. Element nach Anspruch 6, dadurch gekennzeichnet, daß es als Enzym eine Oxidase enthält.

8. Element nach Anspruch 6 oder 7, dadurch gekennzeichnet, daß es als Substanz mit peroxidativer Aktivität Peroxidase enthält.

9. Element nach einem oder mehreren der Ansprüche 6 bis 8, dadurch gekennzeichnet, daß es auf einen pH-Wert von 5 bis 8, vorzugsweise 7 bis 8, abgepuffert ist.

10. Element nach einem oder mehreren der Ansprüche 6 bis 9, dadurch gekennzeichnet, daß es eine Puffersubstanz bestehend aus einer oder mehreren Substanzen der Gruppe : Borat, Citrat, Phosphat, Glutarat, Carbonat oder Tris-Puffersubstanz enthält.

## Claims

1. 2-Hydrazono-4,6-dinitrobenzthiazolones of the formula (I)

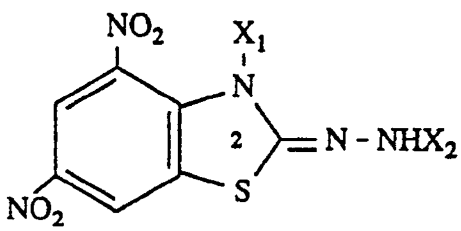

(I)

in which

$X_1$ represents hydrogen, $C_1$-$C_4$-alkyl, $C_1$-$C_4$-hydroxy-alkyl, $C_1$-$C_4$-sulphoalkyl or $C_1$-$C_4$-sulphatoalkyl and $X_2$ represents hydrogen.

2. 2-Hydrazono-4,6-dinitrobenzthiazolones according to Claim 1, in which $X_1$ represents hydrogen or a methyl group.

3. 2-Hydrazono-4,6-dinitrobenzthiazolones according to Claim 1 of the formula (II)

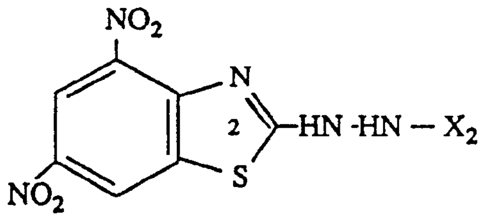

(II)

in which $X_2$ has the meaning given in Claim 1.

4. Use of 2-hydrazono-4,6-dinitrobenzthiazolones according to Claims 1-3 as colour formers in the determination of hydrogen peroxide.

5. Reagent for the determination of hydrogen peroxide in aqueous solutions, containing a buffer substance, a substance having peroxidative activity and 2-hydrazono-4,6-dinitrobenzthiazolones according to Claims 1-3 as colour formers, and also a coupling component.

6. Analytical element for the determination of a species to be analysed in aqueous liquid, having a non-fibrous, isotropically porous diffusion layer and a reagent layer and containing

a) an enzyme which causes the generation of hydrogen peroxide on contact with the species to be analysed, and

b) a reagent for the determination of hydrogen peroxide, comprising

I. a substance having peroxidative activity,

II. a colour former,

III. a coupling component, and, if appropriate,

IV. a buffer substance, characterized in that it contains 2-hydrazono-4,6-dinitrobenzthiazolones according to Claims 1-3 as colour formers.

7. Element according to Claim 6, characterized in that it contains an oxidase as enzyme.

8. Element according to Claim 6 or 7, characterized in that it contains peroxidase as substance with peroxidative activity.

9. Element according to one or more of Claims 6 to 8, characterized in that it is buffered at a pH of 5 to 8, preferably 7 to 8.

10. Element according to one or more of Claims 6 to 9, characterized in that it contains a buffer substance comprising one or more substances from the group comprising : borate, citrate, phosphate, glutarate, carbonate or tris buffer substance.

## Revendications

1. 4,6-dinitrobenzothiazolone-hydrazones-2 de formule (I)

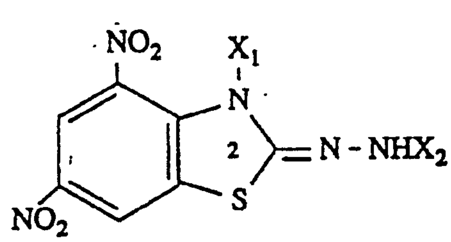

(I)

dans laquelle

$X_1$ représente l'hydrogène, un groupe alkyle en $C_1$-$C_4$, hydroxyalkyle en $C_1$-$C_4$, sulfoalkyle en $C_1$-$C_4$ ou sulfatoalkyle en $C_1$-$C_4$ et

$X_2$ représente l'hydrogène.

2. 4,6-dinitrobenzothiazolone-hydrazones-2 selon la revendication 1, pour lesquelles $X_1$ représente l'hydrogène ou un groupe méthyle.

3. 4,6-dinitrobenzothiazolone-hydrazones-2 selon la revendication 1, répondant à la formule (II)

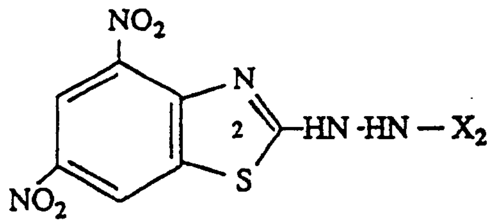

(II)

dans laquelle $X_2$ a la signification indiquée dans la revendication 1.

4. Utilisation des 4,6-dinitrobenzothiazolone-hydrazones-2 selon les revendications 1 à 3, en tant que chromogènes pour l'analyse du peroxyde d'hydrogène.

5. Réactif pour le dosage du peroxyde d'hydrogène dans des solutions aqueuses, contenant une substance tampon, une substance présentant une activité de peroxydation et une 4,6-dinitrobenzothiazolone-hydrazone-2 selon les revendications 1 à 3 en tant que chromogène, ainsi qu'un copulant.

6. Elément d'analyse pour le dosage d'une substance dans un liquide aqueux, comprenant une couche d'étalement non fibreuse, poreuse isotrope et une couche de réactif et contenant

(a) une enzyme qui, au contact avec la substance soumise à l'analyse, provoque la production de peroxyde d'hydrogène et

(b) un réactif pour le dosage du peroxyde d'hydrogène consistant en

I. une substance possédant une activité peroxydante,

II. un chromogène,

III. un copulant et le cas échéant

IV. une substance tampon, caractérisé en ce qu'il contient en tant que chromogène une 4,6-dinitrobenzothiazolone-hydrazone-2 selon les revendications 1 à 3.

7. Elément selon la revendication 6, caractérisé en ce qu'il contient en tant qu'enzyme une oxydase.

8. Elément selon la revendication 6 ou 7, caractérisé en ce qu'il contient en tant que substance ayant une activité peroxydante une peroxydase.

9. Elément selon une ou plusieurs des revendications 6 à 8, caractérisé en ce qu'il est tamponné à un pH de 5 à 8, de préférence de 7 à 8.

10. Elément selon une ou plusieurs des revendications 6 à 9, caractérisé en ce qu'il contient une substance tampon consistant en une ou plusieurs substances du groupe formé par : un borate, un citrate, un phosphate, un glutarate, un carbonate ou du tampon au tris.